# FASCICULE DE BREVET EUROPEEN

(11) **EP 4 051 350 B1**
(45) Date de publication et mention de la délivrance du brevet: **03.01.2024**
(21) Numéro de dépôt: 20789594.7
(22) Date de dépôt: 12.10.2020
(51) Int. Cl.: A61M 16/00, A61M 16/12, F04D 25/06, F04D 25/08, F04D 29/42, F04D 29/58, H02K 9/14, F04D 17/16, H02K 9/06

(54) **APPAREIL MOTORISÉ D'ASSISTANCE RESPIRATOIRE, À DOUBLE REFROIDISSEMENT DU MOTEUR EQUIPANT L'APPAREIL**
MOTORISIERTE ATMUNGSHILFSVORRICHTUNG MIT DOPPELTER KÜHLUNG DES DIE VORRICHTUNG BESTÜCKENDEN MOTORS
MOTORISED RESPIRATORY ASSISTANCE DEVICE, WITH DOUBLE COOLING OF THE MOTOR EQUIPPING THE DEVICE

(30) Priorité: 31.10.2019 FR 1912294
(43) Date de publication de la demande: 07.09.2022
(73) Titulaire: Airfan, 31770 Colomiers (FR)
(72) Inventeur: PRAT, Xavier, 31270 VILLENEUVE TOLOSANE (FR)
(74) Mandataire: Junca, Eric
(86) Numéro de dépôt international: PCT/EP2020/078590
(87) Numéro de publication internationale: WO 2021/083646

(56) Documents cités:
- FR-A1- 2 910 079
- FR-A1- 2 910 081
- FR-A1- 3 057 464

## Description

### DOMAINE TECHNIQUE

L'invention relève du domaine des appareils motorisés de délivrance d'un gaz régulée en pression et en débit, tel que notamment un gaz d'assistance respiratoire. L'appareil de l'invention est un appareil d'assistance respiratoire intégrant un système de double refroidissement par ventilation du moteur équipant l'appareil, tout en réalisant une ségrégation entre le gaz d'assistance respiratoire délivré au patient et un flux d'air de refroidissement du moteur.

Les appareils motorisés de délivrance régulée d'un gaz comprennent communément une enceinte logeant un moteur d'entraînement d'une turbine, tel qu'une roue à ailettes par exemple. La turbine entraînée par le moteur est génératrice d'un flux d'air dont est issu le gaz, par apport au flux d'air d'au moins un adjuvant selon la composition du gaz à délivrer.

L'enceinte ménage aussi un circuit aéraulique à travers lequel circule le flux d'air dont est issu le gaz à délivrer. Le flux d'air circule à travers le circuit aéraulique depuis une entrée d'air, à travers laquelle le flux d'air est admis à l'intérieur de l'appareil, vers une sortie d'air à travers laquelle le flux de gaz est évacué hors de l'appareil.

L'entrée d'air est notamment ménagée par une volute favorisant l'admission et la dynamique du flux d'air à délivrer circulant à travers l'appareil. La volute est alors munie d'un organe de raccordement à un conduit d'admission de l'adjuvant pour son mélange au flux d'air circulant à travers la volute.

Le moteur est plus spécifiquement un moteur électrique asservi permettant de réguler la pression et le débit du flux d'air circulant à travers l'appareil, et par suite de réguler la pression et le débit du gaz délivré par l'appareil.

Une application d'un tel appareil est de fournir une assistance respiratoire à un patient. Le flux d'air prélevé via la turbine est alors enrichi par l'adjuvant, tel que notamment en oxygène. L'oxygène est alors introduit à l'intérieur de la volute pour son mélange au flux d'air et ainsi constituer le gaz à délivrer au patient, désigné par la suite gaz patient.

La motorisation de l'appareil est logée à l'intérieur de l'appareil en évitant au mieux une pollution du gaz patient. La vitesse de rotation du moteur est élevée, à titre indicatif de l'ordre de 80 000 tours/minutes. Le moteur en fonctionnement s'échauffe significativement, et un refroidissement du moteur est à prévoir pour accroître la performance et à la pérennité de l'appareil et/ou pour éviter une élévation en température inopportune du gaz patient.

### ETAT DE LA TECHNIQUE

II a donc été proposé de refroidir le moteur par ventilation, tel qu'il ressort par exemple des documents EP2122180 et WO2018/073275 au nom du présent déposant.

Selon le document EP2122180, l'appareil est pourvu d'une turbine principale génératrice d'un flux d'air principal dont est issu le gaz patient. L'appareil est aussi pourvu d'une turbine annexe génératrice d'un flux d'air de refroidissement du moteur. La turbine principale et la turbine annexe sont montées aux extrémités respectives d'un arbre moteur qui est entraîné par le rotor d'un moteur électrique asservi. Une enceinte ménage un circuit aéraulique comprenant des chemins aérauliques à travers lesquels circulent le flux d'air principal et le flux d'air de refroidissement. Le flux d'air principal est évacué hors de l'appareil après avoir été enrichi en oxygène, et le flux d'air de refroidissement circule autour du bloc moteur pour son refroidissement préalablement à son évacuation hors de l'appareil. Un tel bloc moteur regroupe typiquement les composants du moteur électrique, en étant essentiellement constitué d'un carter logeant un stator et un rotor d'entraînement d'un arbre moteur.

Selon le document WO2018/073275, l'appareil est pourvu d'une turbine unique entraînée par le rotor d'un moteur électrique asservi, la turbine étant génératrice d'un flux d'air dont est issu le gaz patient. Le flux d'air circule à l'intérieur d'une volute, une fraction du flux d'air étant détournée hors de la volute pour refroidir le moteur. Ladite fraction du flux d'air circule entre le rotor et le stator du moteur, puis est évacuée hors de l'appareil à travers une sortie d'air qui lui est affectée en interdisant son renvoi dans le gaz patient évacué hors de l'appareil.

Cependant, les solutions proposées par l'art antérieur ne sont pas pleinement satisfaisantes et méritent d'être améliorées pour répondre au mieux aux exigences structurelles et fonctionnelles de tels appareils d'assistance respiratoire.

Il est plus particulièrement constaté que les solutions proposées par l'art antérieur pour refroidir le moteur équipant l'appareil ne permettent pas de fournir une ventilation du moteur sur de larges gammes de régulation du débit et/ou de la pression du ou des flux d'air circulant à travers l'appareil. Ceci présente l'inconvénient que certaines plages de fonctionnement du moteur induisent son échauffement conséquent et par suite une réduction de sa durée de vie.

Il en résulte que des plages potentielles de fonctionnement du moteur sont proscrites, notamment concernant un fonctionnement du moteur procurant un flux de gaz patient à faible débit et forte pression, qui sont par exemple particulièrement utiles pour un usage néonatal ou un usage pédiatrique.

### EXPOSE DE L'INVENTION

L'invention a pour objet un appareil motorisé d'assistance respiratoire intégrant un système de refroidissement par ventilation du moteur équipant l'appareil.

Le but de l'invention est de procurer un refroidissement du moteur équipant l'appareil, qui permette son utilisation sur une vaste gamme de conditions de fonctionnement, y compris sur des plages de fonctionnement extrêmes telles que par exemple pour la délivrance d'un flux de gaz patient à faible débit et forte pression.

Un objectif de l'invention est de procurer un refroidissement du moteur qui soit particulièrement efficace, afin de limiter son échauffement en fonctionnement et ainsi accroître sa durée de vie. Il est notamment visé l'obtention d'un refroidissement optimisé du moteur permettant de le préserver d'une dégradation résultant d'un échauffement conséquent du moteur en fonctionnement, tout en conservant durablement et en toute fiabilité les performances du moteur.

Un objectif connexe de l'invention est de procurer un refroidissement du moteur sans affecter la qualité du gaz patient. Il est notamment visé d'organiser les modalités de refroidissement du moteur en évitant de souiller le gaz délivré au patient par suite de sa ventilation.

Un autre objectif connexe de l'invention est de limiter l'encombrement et/ou la masse de l'appareil, en particulier en l'équipant d'un système de refroidissement du moteur qui soit simple et peu encombrant. Il est notamment visé:
- de limiter le nombre de composants de l'appareil et de faciliter leur obtention et/ou leur assemblage sur l'appareil, sans affecter la qualité obtenue de l'appareil ni sa pérennité et/ou sans affecter ses performances, et/ou
- de faciliter l'intégration du système de refroidissement à l'intérieur de l'appareil et/ou d'éviter d'encombrer l'espace environnant une volute équipant l'appareil, à travers laquelle volute circule le flux d'air dont est issu le gaz patient. Il est notamment recherché de dégager l'environnement de la volute pour faciliter son raccordement à un conduit d'admission d'au moins un adjuvant, dont notamment au moins de l'oxygène, et/ou à un conduit d'adduction du gaz patient vers le patient.

Un autre objectif de l'invention est de permettre une utilisation de l'appareil avec une large plage d'apport en quantité souhaitée d'au moins un adjuvant au gaz patient, tel que de l'oxygène à forte concentration pouvant s'étendre jusqu'à 100%. Il est notamment visé d'organiser l'appareil de manière à préserver le moteur d'une mise en contact avec ledit au moins adjuvant qui est potentiellement agressif pour le moteur tel que notamment l'oxygène, pour le protéger d'une oxydation ou plus généralement d'une attaque chimique par l'adjuvant.

Un autre objectif de l'invention est de pouvoir effectuer des opérations de maintenance potentiellement soutenues de l'appareil pour son nettoyage et/ou son assainissement par au moins un produit désinfectant réputé agressif. Il est dès lors à éviter une dégradation des composants de l'appareil par le produit désinfectant, notamment en ce qui concerne le moteur.

Pour ce faire, il est proposé par l'invention de refroidir le moteur par un même flux d'air de refroidissement d'abord interne puis externe au moteur, tout en réalisant une ségrégation entre ce flux de refroidissement et le flux d'air principal dont est issu le gaz patient par une organisation indépendante des chemins aérauliques suivis par ces flux. Le refroidissement intérieur s'effectue entre le stator et le rotor du moteur, alors que le refroidissement extérieur au moteur s'établit autour du carter qui loge le moteur.

Un tel double refroidissement est alors exclusivement fourni par le flux d'air de refroidissement du moteur qui circule successivement à l'intérieur du moteur entre le rotor et le stator, puis à l'extérieur du moteur autour du carter enveloppant le moteur. Le refroidissement du moteur est ainsi optimisé par suite des échanges thermiques successifs correspondants.

La ségrégation entre le chemin aéraulique annexe et le chemin aéraulique principal est formée par un cloisonnement entre lesdits chemins. Les fuites d'air s'infiltrant dans l'espace dédiée à la libre rotation de l'arbre moteur sont collectées entre le bloc moteur et ledit cloisonnement. Les fuites d'air issues du flux d'air de refroidissement et les fuites d'air issues du flux d'air principal sont alors drainées par le flux d'air de refroidissement circulant autour du bloc moteur, jusqu'à leur évacuation hors de l'appareil conjointement avec le flux d'air de refroidissement.

Plus précisément, l'invention propose un appareil motorisé d'assistance respiratoire à système de refroidissement intégré, du type d'appareil comportant une enceinte ménageant un compartiment délimité par une chemise logeant un bloc moteur. Le bloc moteur comporte un carter de réception au moins d'un rotor et d'un stator constitutifs d'un moteur d'entraînement d'au moins deux turbines montées aux extrémités axiales respectives d'un arbre moteur entraîné par le rotor.

Lesdites turbines se composent au moins d'une turbine principale génératrice d'un flux d'air principal dont est issu un gaz patient et d'une turbine annexe génératrice d'un flux d'air de refroidissement du moteur. L'enceinte ménage un circuit aéraulique composé au moins de deux chemins aérauliques se composant d'au moins un chemin aéraulique principal d'acheminement du flux d'air principal et d'au moins un chemin aéraulique annexe d'acheminement du flux d'air de refroidissement et comportant chacun une entrée d'air des turbines vers l'intérieur de l'appareil et une sortie d'air vers l'extérieur de l'appareil.

Dans ce type d'appareil l'invention propose de segmenter le chemin aéraulique annexe en deux portions placées successivement en communication aéraulique l'une avec l'autre, dont une portion interne au moteur et une portion externe entourant le bloc moteur. Ainsi dans un appareil du type susvisé, le chemin aéraulique annexe comporte des portions interne et externe dans lesquels le flux de refroidissement circule successivement en sens opposé: une portion interne de circulation du flux d'air de refroidissement provenant de l'entrée d'air correspondante et qui s'étend à l'intérieur du moteur entre le stator et le rotor; et une portion externe qui s'étend, parallèlement à la portion interne, à l'intérieur d'un espace annulaire ménagé autour du bloc moteur entre le carter du bloc moteur et la chemise de l'enceinte jusqu'à la sortie d'air correspondante ménagée dans cette chemise du côté de l'entrée d'air de refroidissement.

De plus, le chemin aéraulique principal et le chemin aéraulique annexe sont séparés l'un de l'autre par un cloisonnement ménagé entre eux, ledit cloisonnement dissociant la circulation du flux d'air principal et la circulation du flux d'air de refroidissement à l'intérieur de l'appareil. Le flux d'air principal est alors exclusivement dédié à la délivrance du gaz patient et le flux d'air de refroidissement exclusivement dédié au double refroidissement du moteur.

Une telle architecture du chemin aéraulique annexe procure un double refroidissement du moteur par le flux d'air de refroidissement, dont un refroidissement interne au moteur entre le rotor et le stator puis un refroidissement externe du moteur autour du carter qui le loge. Le refroidissement du moteur est obtenu performant à partir de sa double ventilation par le flux d'air de refroidissement seulement. Un passage du flux d'air principal à travers le moteur est ainsi interdit pour éviter sa détérioration par un adjuvant délétère - oxygène notamment - qui est contenu dans le gaz patient et/ou par un agent de nettoyage et/ou d'assainissement lors d'une opération de maintenance de l'appareil. Parallèlement, une pollution du flux d'air principal par le flux d'air de refroidissement est également évitée.

L'optimisation du refroidissement du moteur ainsi obtenu permet de le préserver et d'accroître sa durée de vie, et cela en permettant son fonctionnement y compris selon des plages extrêmes de régulation du flux d'air principal en pression et en débit, tel que notamment des plages de régulation à faible débit et forte pression du flux d'air principal pour un usage néonatal ou un usage pédiatrique de l'appareil par exemple.

L'affectation du flux d'air de refroidissement exclusivement au refroidissement du moteur et du flux d'air principal exclusivement à l'assistance respiratoire d'un patient, permet d'éviter un souillage du flux d'air principal par le flux d'air de refroidissement par ségrégation entre le chemin aéraulique principal et le chemin aéraulique secondaire, tel que notamment via un cloisonnement ménagé entre eux pouvant être de structure simple.

Les circulations forcées par les turbines respectivement du flux d'air principal et du flux d'air de refroidissement vers l'extérieur de l'appareil sont ségréguées. Des fuites d'air respectivement issues du flux d'air principal et du flux d'air de refroidissement sont alors collectées et mélangées dans une chambre qui leur est dédiée et qui est en communication aéraulique avec la portion externe du chemin aéraulique annexe. Les fuites d'air collectées et mélangées sont alors drainées par le flux d'air de refroidissement circulant autour du bloc moteur, et sont évacuées hors de l'appareil sans risque de dégradation du moteur.

De préférence, un volume interfacé entre le bloc moteur et ledit cloisonnement ménage une chambre de collecte et de mélange entre, d'une part de fuites d'air issues du flux d'air principal et provenant du chemin aéraulique principal en traversant le dit cloisonnement et, d'autre part, de fuites d'air issues du flux d'air de refroidissement et provenant du bloc moteur. La chambre est en communication aéraulique via au moins un passage d'air avec la portion externe du chemin aéraulique annexe, de sorte que lesdites fuites d'air sont drainées hors de la chambre, notamment sous forme d'un flux de fuites d'air mélangées, vers l'intérieur de la portion externe du chemin aéraulique annexe par le flux d'air de refroidissement évacué hors du bloc moteur. Le flux d'air de refroidissement et lesdites fuites d'air sont alors conjointement évacués hors de l'appareil via la sortie d'air du chemin aéraulique annexe.

Avantageusement, le flux d'air de refroidissement est admis à l'intérieur de la portion interne du chemin aéraulique annexe, puis est évacué hors du bloc moteur vers la portion externe du chemin aéraulique annexe à travers au moins une deuxième ouverture traversant la paroi du carter du bloc moteur.

Selon une forme de réalisation, ladite au moins deuxième ouverture traverse radialement la paroi du carter du bloc moteur et est disposée à faible distance axiale du cloisonnement, étant compris au plus proche du cloisonnement. Il est aussi compris que les notions relatives axiale et radiale sont identifiées au regard de la direction d'extension axiale de l'arbre moteur à l'intérieur de l'appareil. La chambre est axialement ménagée entre le bloc moteur et le cloisonnement en débouchant axialement sur la portion externe du chemin aéraulique annexe.

Ceci permet d'optimiser l'extension axiale de la portion externe du chemin aéraulique annexe. Ceci permet aussi de favoriser l'entraînement des fuites d'air hors de la chambre via ledit au moins un passage d'air - par le flux d'air de refroidissement évacué hors du bloc moteur - vers la portion externe du chemin aéraulique annexe.

Afin de maximiser la réduction de l'infiltration des fuites d'air, des organes d'étanchéité en matériau polymère thermostable sont montés autour de l'arbre moteur avec un jeu minimal, et sont composés au moins d'un organe d'étanchéité solidaire du cloisonnement du côté de la volute, et d'organes d'étanchéité solidaires de paliers de roulement montés sur le carter hors du bloc moteur.

Selon une forme de réalisation, la turbine annexe est logée à l'intérieur d'une cavité d'admission du flux d'air de refroidissement vers l'intérieur du bloc moteur, via l'entrée d'air du chemin aéraulique annexe. La cavité est isolée de la portion externe du chemin aéraulique annexe et est ouverte sur l'intérieur du bloc moteur via au moins un passage d'air ménagée à travers le carter.

La cavité logeant la turbine annexe contraint le flux d'air de refroidissement admis à l'intérieur de l'appareil à circuler vers la portion interne du chemin aéraulique annexe, préalablement à la circulation du flux d'air de refroidissement à travers la portion externe du chemin aéraulique annexe.

Notamment, selon une forme de réalisation, la cavité est délimitée entre la paroi périphérique de la chemise et une couronne entourant radialement le carter du bloc moteur. La sortie d'air du chemin aéraulique annexe est ménagée radialement à travers la chemise en interposition axiale entre la couronne et ladite au moins une deuxième ouverture traversant la paroi du carter du bloc moteur. Suivant l'extension axiale de l'appareil, la sortie d'air du chemin aéraulique annexe est notamment ménagée au plus proche de la couronne et à distance significative d'éloignement de la deuxième ouverture, pour accroître au mieux l'extension de la portion externe du chemin aéraulique annexe.

Par exemple encore selon une autre forme de réalisation, la cavité est ménagée par le volume intérieur d'un caisson qui est monté axialement sur le carter et qui ménage l'entrée d'air du chemin aéraulique annexe. Le caisson est disposé à l'intérieur du compartiment recevant le bloc moteur en prolongement axial du carter. Le caisson est notamment rapporté et fixé au carter par scellement étanche entre le caisson et le carter.

Selon une forme de réalisation, le caisson est configuré en cône dont le plus grand débouché est orienté vers le moteur et dont le plus petit débouché est prolongé axialement par un conduit d'entrée d'air intégré au caisson. Le conduit d'entrée d'air traverse axialement par emboîtement une paroi de la chemise, en ménageant l'entrée d'air du chemin aéraulique annexe et en formant un organe de centrage axial du bloc moteur à l'intérieur du compartiment.

Selon une forme de réalisation, la sortie d'air du chemin aéraulique annexe est ménagée par le débouché vers l'extérieur de l'enceinte d'un conduit de sortie d'air intégré à la chemise.

Selon une forme de réalisation, le conduit d'entrée d'air et le conduit de sortie d'air du chemin aéraulique annexe sont disposés à une première extrémité axiale de l'appareil qui est opposée à son autre deuxième extrémité axiale à laquelle le chemin aéraulique principal est ménagé entre l'entrée d'air et la sortie d'air qu'il comporte.

Selon une forme de réalisation, l'enceinte comprend une volute logeant la turbine principale et délimitant le chemin aéraulique principal. Ladite volute ménage l'entrée d'air et la sortie d'air du chemin aéraulique principal et peut être pourvue d'au moins un organe de raccordement à un conduit d'admission d'un adjuvant à l'intérieur de la volute.

Selon une forme de réalisation, au moins une paroi délimitant la volute ménage le cloisonnement de séparation entre le chemin aéraulique principal et le chemin aéraulique annexe. Ladite paroi ménageant le cloisonnement incorpore ledit organe de raccordement et délimite entre eux d'une part la volute et la sortie d'air du flux d'air principal hors de l'appareil qui est incorporée à la volute, et d'autre part le compartiment de réception du bloc moteur.

### PRESENTATION DES FIGURES

L'invention sera mieux comprise à la lecture de la description détaillée qui suit d'exemples de réalisation de l'invention, en relation avec les figures suivantes des planches annexées qui représentent:
- [Fig.1] La figure, une illustration en coupe axiale d'un appareil motorisé d'assistance respiratoire à système de refroidissement intégré, conforme à un premier exemple de réalisation l'invention;
- [Fig.2] La figure 2, une illustration en coupe axiale d'un appareil motorisé d'assistance respiratoire à système de refroidissement intégré, conforme à un deuxième exemple de réalisation l'invention;
- [Fig.3] La figure 3, un agrandissement (A) de la figure 1, illustrant en coupe axiale des modalités de gestion de fuites d'air circulant à travers l'appareil représenté sur la figure 1, et
- [Fig.4] La figure 4, un agrandissement (B) de la figure 2, illustrant en coupe axiale des modalités de gestion de fuites d'air circulant à travers l'appareil représenté sur la figure 2.

### DESCRIPTION DETAILLEE

Les figures et leur description détaillée non limitative, exposent l'invention selon des modalités particulières qui ne sont pas restrictives quant à la portée de l'invention telle que définie par les revendications. Les figures et leur description détaillée peuvent servir à mieux appréhender et définir l'invention, si besoin en relation avec la description générale qui vient d'en être faite.

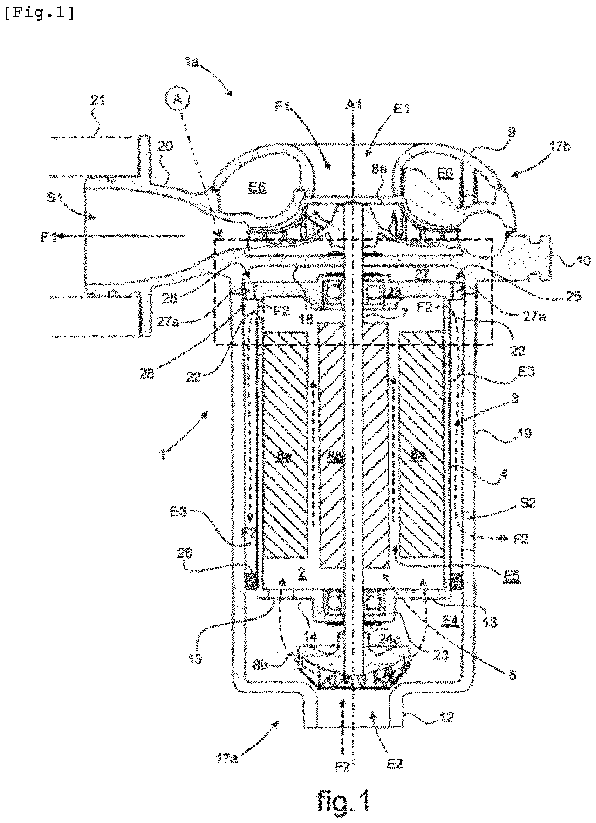

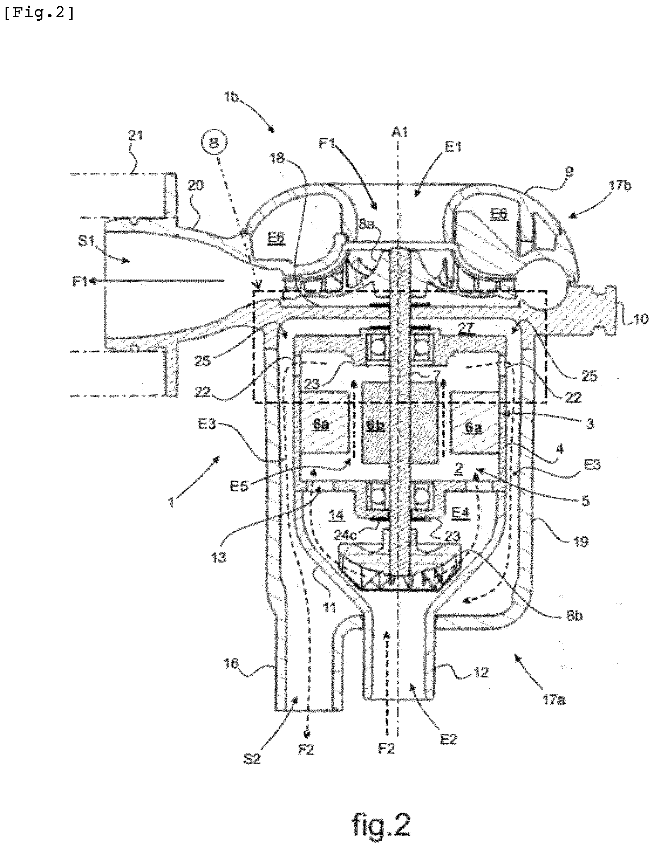

Sur la figure 1 et la figure 2, des appareils 1a, 1b motorisés de délivrance régulée d'un gaz sont appliqués à l'assistance respiratoire d'un patient. Les appareils 1a, 1b présentent une extension axiale A1 et ménagent une enceinte 1 formée essentiellement par une volute 9 et par une chemise 19 assemblées axialement entre elles. La chemise 19 délimite à l'intérieur des appareils 1a, 1b un compartiment 2 de réception d'un bloc moteur 3. Le bloc moteur 3 comprend un carter 4 logeant un moteur 5 électrique, comprenant un stator 6a et un rotor 6b d'entraînement en rotation d'un arbre moteur 7, et monté sur des paliers de roulement 23 autour de cet arbre moteur. Il est compris que l'extension axiale des appareils 1a, 1b est identifiée par l'orientation de l'axe A1 d'extension de l'arbre moteur 7.

Les appareils 1a, 1b sont organisés pour générer deux flux d'air F1, F2. Un flux d'air principal F1 est dédié à l'assistance respiratoire d'un patient et un flux d'air de refroidissement F2 est dédié au refroidissement du moteur 5 par suite de sa mise en fonctionnement. A cet effet, des turbines 8a, 8b sont montées aux extrémités axiales respectives de l'arbre moteur 7, en étant par exemple chacune formées d'au moins une roue à ailettes.

Une turbine annexe 8b génératrice du flux d'air de refroidissement F2 est montée à une première des extrémités axiales de l'arbre moteur 7. Une turbine principale 8a génératrice du flux d'air principal F1 est montée à l'autre deuxième des extrémités axiales de l'arbre moteur 7. Par suite d'une activation régulée du moteur 5, l'arbre moteur 7 est entraîné en rotation via le rotor 6b du moteur 5. La mise en rotation de l'arbre moteur 7 permet d'entraîner la turbine principale 8a en rotation pour générer le flux d'air principal F1 et la turbine annexe 8b pour générer le flux d'air de refroidissement F2.

La turbine principale 8a est logée à l'intérieur d'une volute 9 que comprend l'enceinte 1. La volute 9 ménage une première entrée d'air E1 pour l'admission du flux d'air principal F1 à l'intérieur des appareils 1a, 1b et une première sortie d'air S1 pour l'évacuation à l'extérieur des appareils 1a, 1b d'un gaz patient issu du flux d'air principal F1 qui est enrichi en oxygène. L'oxygène est introduit dans le flux d'air principal F1 à l'intérieur de la volute 9, via un conduit d'admission de l'oxygène (non représenté) à l'intérieur de l'enceinte 1. De plus, une interface de suspension 10 de la turbine principale 8a atténue les vibrations et les bruits.

Un chemin aéraulique principal E1, E6, S1 d'acheminement du flux d'air principal F1 à l'intérieur des appareils 1a, 1b est délimité par le volume intérieur E6 de la volute 9 qui s'étend en interface entre la première entrée d'air E1 et la première sortie d'air S1 ménagées par la volute 9. Le chemin aéraulique principal E1, E6, S1 est délimité par l'enveloppe extérieure de la volute 9 et par un cloisonnement 18 qui est intégrée à la volute 9. Le cloisonnement 18 isole le volume intérieur E6 de la volute 9 par rapport au compartiment 2 de réception du bloc moteur 3. Le gaz patient est délivré au patient pour lui fournir une assistance respiratoire, via une conduite 21 raccordée à un embout 20 qui est intégré à la volute 9 et qui ménage la première sortie d'air S1.

La turbine annexe 8b est logée à l'intérieur d'une cavité E4 d'admission du flux d'air de refroidissement F2 vers l'intérieur des appareils 1a, 1b. La cavité E4 est ouverte sur une deuxième entrée d'air E2 d'admission du flux d'air de refroidissement à l'intérieur des appareils 1a, 1b. La deuxième entrée d'air E2 est ménagée par le débouché vers l'extérieur de l'appareil 1a, 1b d'un conduit d'entrée d'air 12 coaxial à l'arbre moteur 7. La cavité E4 est aussi ouverte sur le volume intérieur du bloc moteur 3, via des premières ouvertures 13 d'orientation axiale qui sont ménagées à travers la paroi du carter 4 ménageant une cloison de séparation 14 axiale entre la cavité E4 et le moteur 5.

Le flux d'air de refroidissement F2 admis à l'intérieur des appareils 1a, 1b via la deuxième entrée d'air E2, circule à l'intérieur de la cavité E4 puis est admis à l'intérieur du volume intérieur du bloc moteur 3 via les premières ouvertures 13. Un premier refroidissement interne au moteur 5 est ainsi réalisé par le flux d'air de refroidissement F2 s'écoulant entre le rotor 6b et le stator 6a du moteur 5.

Puis le flux d'air de refroidissement F2 est évacué hors du bloc moteur 3 à travers des deuxièmes ouvertures 22 radiales que comporte la paroi du carter 4 dans sa zone d'extension axiale. Le flux d'air de refroidissement F2 est alors admis à l'intérieur d'un espace annulaire E3 entourant le bloc moteur 3 qui est ménagé à l'intérieur du compartiment 2 entre la chemise 19 et la paroi périphérique du carter 4.

Le flux d'air de refroidissement F2 circule alors autour du bloc moteur 3 jusqu'à son évacuation hors des appareils 1a, 1b via une deuxième sortie d'air S2 que comporte la chemise 19. Un deuxième refroidissement externe du moteur 5 est ainsi réalisé par le flux d'air de refroidissement F2 s'écoulant à l'intérieur du compartiment 2 autour du bloc moteur 3.

Un chemin aéraulique annexe E2, E4, 13, E5, 22, E3, S2 d'acheminement du flux d'air de refroidissement F2 à l'intérieur des appareils 1a, 1b est ainsi délimité à l'intérieur compartiment 2. Le chemin aéraulique annexe E2, E4, 13, E5, 22, E3, S2, s'étend plus spécifiquement depuis la deuxième entrée d'air E2 vers la deuxième sortie d'air S2, en comportant successivement - suivant le sens de circulation du flux d'air de refroidissement F2 à l'intérieur des appareils - la cavité E4, les premières ouvertures 13, le volume intérieur du bloc moteur 3, les deuxièmes ouvertures 22 et l'espace annulaire E3 entourant le bloc moteur 3.

Plus particulièrement, le chemin aéraulique annexe comporte une portion interne E5 formée par le volume intérieur du moteur 5 et une portion externe E2, E4, 13, 22, E3, S2 extérieure au moteur 5. Ladite portion externe se compose de la deuxième entrée d'air E2, de la cavité E4, des premières ouvertures 13, des deuxièmes ouvertures 22, de l'espace annulaire E3 entourant le bloc moteur 3 et de la deuxième sortie d'air S2. Pour accroître au mieux le refroidissement externe du moteur 5, la circulation du flux d'air de refroidissement F2 autour du bloc moteur 3 est optimisée. Les deuxièmes ouvertures 22 sont ménagées radialement à travers la paroi périphérique du carter 4 à faible distance axiale du cloisonnement 18 et à large distance axiale de la deuxième sortie d'air S2 du chemin aéraulique annexe E2, E4, 13, E5, 22, E3, S2.

Le refroidissement du moteur 5 est ainsi obtenu performant par la double circulation du flux d'air de refroidissement F2 en premier lieu à l'intérieur du moteur 5 entre le rotor 6b et le stator 6a puis en deuxième lieu autour du bloc moteur 3 préalablement à son évacuation hors des appareils 1a, 1b. Le flux d'air de refroidissement F2 est spécifiquement dédié au refroidissement du moteur 5 en étant prélevé depuis l'environnement extérieur des appareils 1a, 1b, qui est exempt d'agent susceptible d'être délétère pour les composants du moteur 5.

Le flux d'air principal F1 est propulsé par la turbine principale 8a à l'intérieur de la volute 9 depuis la première entrée d'air E1 vers la première sortie d'air S1. Le flux d'air de refroidissement F2 est propulsé par la turbine annexe 8b depuis la deuxième entrée d'air E2 à l'intérieur puis à l'extérieur du bloc moteur 3 vers la deuxième sortie d'air S2.

Le cloisonnement 18 interfacé entre la volute 9 et le compartiment 2 logeant le bloc moteur 3, procure une ségrégation entre les chemins respectifs - chemin aéraulique principal et chemin aéraulique annexe - de circulations forcées du flux d'air principal F1 et du flux d'air de refroidissement F2 à travers les appareils 1a, 1b. Une telle ségrégation procure un refroidissement intérieur et extérieur efficace du moteur 5, tout en évitant un passage vers l'intérieur du compartiment 2 du flux d'air principal F1 dont la circulation est forcée seulement à l'intérieur de la volute 9. Inversement, il est aussi évité un passage vers l'intérieur de la volute 9 du flux d'air de refroidissement F2 dont la circulation est forcée seulement à l'intérieur du compartiment 2.

Il en ressort qu'il est évité que ne se produise une dégradation du moteur 5 pouvant être induite par un échauffement excessif du moteur 5 et/ou par un agent délétère acheminé par le flux d'air principal F1 - oxygène ou agent d'entretien des appareils1a, 1b notamment - dont la circulation est forcée seulement à travers la volute 9. Inversement, il est aussi évité un souillage du flux d'air principal F1 pouvant être induit par une pollution éventuelle du flux d'air de refroidissement F2 par suite de son passage à l'intérieur du bloc moteur 3, la circulation forcée du flux d'air de refroidissement F2 s'effectuant seulement à l'intérieur du compartiment 2.

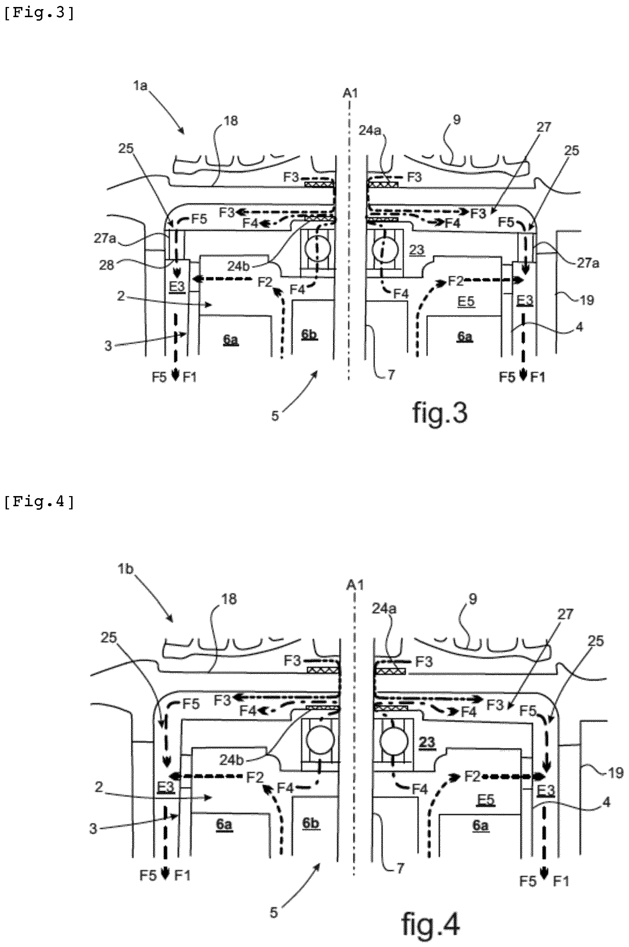

Plus particulièrement visible sur la figure 3 et la figure 4, il est aussi tenu compte du fait que l'arbre moteur 7 traverse le cloisonnement 18 et le carter 4 du bloc moteur 3. Des fuites d'air F3 issues du flux d'air principal F1 peuvent s'infiltrer entre l'arbre moteur 7 et le cloisonnement 18 depuis la volute 9 vers le compartiment 2. Inversement des fuites d'air F4 issues du flux d'air de refroidissement F2 peuvent s'infiltrer entre l'arbre moteur 7 et le cloisonnement 18 depuis le compartiment 2 vers la volute 9.

Il est à éviter que les fuites d'air F3 issues du flux d'air principal F1 ne dégradent le moteur 3, et/ou que les fuites d'air F4 issues du flux d'air de refroidissement F2 ne polluent le flux d'air principal F1.

Pour conforter la rigueur de la ségrégation entre le chemin aéraulique principal E1, E6, S1 et la portion interne E5 du chemin aéraulique annexe, les fuites d'air F3, F4 sont collectées et drainées vers l'extérieur des appareils 1a, 1b en interdisant leur passage à l'intérieur du bloc moteur 3.

Plus particulièrement, les fuites d'air F3, F4 s'infiltrant entre l'arbre moteur 7 et le cloisonnement 18 et/ou le carter 4 sont collectées à l'intérieur d'une chambre 27 s'étendant axialement entre le bloc moteur 3 et le cloisonnement 18. Les fuites d'air F3 en provenance de la volute 9 et les fuites d'air F4 en provenance du bloc moteur 3 se mélangent alors à l'intérieur de la chambre 27. La chambre 27 est en communication aéraulique avec l'espace annulaire E3 entourant le bloc moteur 3, via au moins un passage d'air 25, de sorte que le mélange des fuites d'air F3, F4 est entraîné - sous forme d'un flux de fuites d'air mélangées F5 - par le flux d'air de refroidissement F2 évacué hors du bloc moteur 3, via les deuxièmes ouvertures 22, et circulant autour du bloc moteur 3.

Les fuites d'air F3, F4 sont alors mêlées au flux d'air de refroidissement F2 circulant à l'extérieur du bloc moteur 3 sans risque de mise en contact entre les fuites d'air F3, F4 et le moteur 5. Les fuites d'air F3, F4 et le flux d'air de refroidissement F2 sont alors évacués conjointement hors des appareils 1a, 1b via la deuxième sortie d'air S2.

Afin de réduire au maximum l'infiltration desdites fuites d'air F3, F4 entre la volute 9 et le compartiment 2, des organes d'étanchéité en matériau polymère 24a, 24b, 24c (figures 1 et 2) - comme par exemple des rondelles en élastomère ou en silicone ou autre polymère thermostable - sont avantageusement prévus. Les organes d'étanchéité 24a, 24b, 24c sont montés autour de l'arbre moteur 7 avec un jeu minimal. Un premier organe d'étanchéité 24a est solidarisé au cloisonnement 18 du côté et vers l'intérieur de la volute 9.

Des deuxième et troisième organe d'étanchéité 24b, 24c sont placés à l'extérieur du bloc moteur 3 et solidarisés sur les paliers de roulement 23 pour faire obstacle à une échappée hors du bloc moteur 3 d'un lubrifiant, couramment présent dans les paliers de roulement 23 interfacés entre l'arbre moteur 7 et le carter 4 du bloc moteur 3.

Selon la forme de réalisation de l'appareil 1a illustré sur la figure 1, la cavité E4 logeant la turbine annexe 8b est formée d'un volume intérieur du compartiment 2, qui s'étend en interposition axiale entre le bloc moteur 3 et la deuxième entrée d'air E2. La cavité E4 est délimitée entre la paroi de la chemise 19 et une couronne 26 entourant radialement le carter 4 du bloc moteur 3 à son extrémité orientée vers la deuxième entrée d'air E2. La couronne 26 s'étend diamétralement entre le carter 4 et la paroi de la chemise 19 dans sa partie d'extension axiale, en forçant le passage du flux d'air de refroidissement F2 à l'intérieur du bloc moteur 3 et en faisant obstacle à un passage autour du bloc moteur 3 du flux d'air de refroidissement F2 admis à l'intérieur du compartiment 2.

Le conduit d'entrée d'air 12 est formé par la paroi de la chemise 19. La deuxième sortie d'air S2 est ménagée radialement à travers la chemise 19 à proximité axiale de la couronne 26 et en éloignement axial des deuxièmes ouvertures 22 traversant la paroi du carter 4, pour optimiser le refroidissement externe du moteur 3 lors de la circulation du flux d'air de refroidissement F2 autour du bloc moteur 3. La deuxième sortie d'air S2 peut être ménagée par un conduit de sortie d'air d'extension radiale intégré à la chemise 19.

En se reportant aussi à la figure 3, plusieurs passages d'air 25 d'évacuation des fuites d'air F3, F4 hors de la chambre 27 - sous forme du flux de fuites d'air mélangées F5 - sont formés d'une pluralité de canaux 27a. Lesdits canaux 27a sont ménagés axialement à travers la paroi du carter 4 dans sa partie orientée vers la chambre 27 qui s'étend diamétralement entre la paroi périphérique de la chemise 19 et la paroi périphérique du carter 4. Plus particulièrement, les canaux 27a sont répartis radialement à travers une portion annulaire 28 de la paroi du carter 4 qui s'étend diamétralement à l'intérieur de l'espace annulaire E3 entourant le bloc moteur 3.

Selon la forme de réalisation de l'appareil 1b illustré sur la figure 2, la cavité E4 logeant la turbine annexe 8b est formée du volume intérieur d'un caisson 11 logé à l'intérieur du compartiment 2 en étant axialement solidarisé au carter 4 du bloc moteur 3. Le caisson 11 est agencé en entonnoir, en comportant un cône axialement prolongé par le conduit d'entrée d'air 12 via lequel le flux d'air de refroidissement F2 est admis à l'intérieur de l'appareil 1b.

Le conduit d'entrée d'air 12 traverse par emboîtement étanche la paroi de l'enceinte 1 en formant un organe de centrage du bloc moteur 3 à l'intérieur du compartiment 2. Un conduit de sortie d'air 16 est ménagé par la paroi de la chemise 19 en étant orienté suivant l'orientation axiale de l'appareil 1b. Le conduit de sortie d'air 16 débouche hors de l'enceinte 1 pour ménager la deuxième sortie d'air S2, via laquelle le flux d'air de refroidissement F2 est évacué hors de l'appareil 1b.

En se reportant aussi à la figure 4, le passage d'air 25 d'évacuation des fuites d'air F3, F4 hors de la chambre 27 est formé par un débouché sur la chambre 27 de l'espace annulaire E3 entourant le bloc moteur 3. L'entraînement des fuites d'air F3, F4 mélangées à l'intérieur de la chambre 27 par le flux d'air de refroidissement F2 circulant autour du bloc moteur 3 en est favorisé.

L'invention n'est pas limitée aux exemples décrits et représentés. Ainsi, divers aménagements complémentaires de l'appareil 1a, 1b relevant de l'invention peuvent être prévus.

Par exemple, le flux d'air de refroidissement F2 peut être évacué via plusieurs deuxièmes sorties d'air équipant les appareils 1a, 1b, pour accroître le débit et/ou la vélocité du flux d'air de refroidissement F2 circulant à travers les appareils 1a, 1b.

Plus particulièrement concernant l'appareil 1a représenté sur la figure 1, plusieurs deuxièmes sorties d'air S2 peuvent par exemple être ménagées radialement à travers la paroi de la chemise 19. Subsidiairement, la ou les deuxièmes sorties d'air peuvent être chacune ménagées par un conduit de sortie d'air 16 intégré à la chemise 19.

En outre, concernant l'appareil 1b représenté sur la figure 2, plusieurs deuxièmes sorties d'air S2 peuvent par exemple être ménagées axialement à travers la paroi de la chemise 19 à la première extrémité axiale 17a de l'appareil1b. Les deuxièmes sorties d'air S2 sont alors par exemple formées de trouées d'extension axiale réparties radialement à travers la paroi de la chemise 19, en étant chacune exemptes d'un dit deuxième conduit de sortie d'air 16.

Par ailleurs, l'espace annulaire E3 qui entoure le bloc moteur 3 et qui participe de la portion externe du chemin aéraulique annexe via laquelle le flux d'air de refroidissement F2 circule autour du bloc moteur 3, peut loger un ou plusieurs éléments d'échange thermique entre le flux d'air de refroidissement F2 et le carter 4 du bloc moteur 3 et/ou la chemise 19. De tels éléments d'échange thermique sont par exemple composés d'ailettes ajourées ou par exemple encore sont ménagés par l'armature d'un treillis métallique. Les éléments d'échange thermique sont notamment répartis axialement à l'intérieur de l'espace annulaire E3 entourant le bloc moteur 3 et participant du chemin aéraulique annexe, en étant placés en contact avec le carter 4 du bloc moteur 3 et avec la chemise 19.

En outre, l'espace annulaire E3 entourant le bloc moteur 3 via lequel le flux d'air de refroidissement F2 circule autour du bloc moteur 3, peut être configuré en chicane. Une telle chicane permet d'accroître le chemin parcouru par le flux d'air de refroidissement F2 autour du bloc moteur 3. En outre, une telle chicane peut être avantageusement composée de dits éléments à échange thermique tel que précédemment visés.

L'appareil 1a, 1b peut être équipé d'un mécanisme de débrayage de l'entraînement de la turbine principale 8a par l'arbre moteur 7. Un tel mécanisme de débrayage permet, en cas d'un arrêt temporaire de la délivrance du gaz patient, de maintenir l'entraînement de la turbine annexe 8b par le moteur 5. L'entraînement de la turbine annexe 8b seulement, peut être réalisé sans soumettre le moteur 5 à une charge conséquente pour maintenir son refroidissement par le flux d'air de refroidissement F2 jusqu'à un seuil de refroidissement prédéfini.

Un maintien du refroidissement du moteur 5 en cas d'arrêt de la délivrance du gaz patient permet d'achever rapidement le refroidissement du moteur 5, sous dépendance par exemple d'une temporisation et/ou d'un capteur de température logé dans le bloc moteur 3 et mesurant la température du moteur 5. Le mécanisme de débrayage peut par exemple être placé à l'intérieur du bloc moteur 3, en étant configuré en un mécanisme de crabotage placé sur l'arbre moteur 7.

Le mécanisme de crabotage peut être du type activable par une commande électromagnétique de manoeuvre du crabot qu'il comporte, entre une position active d'embrayage et une position inactive de débrayage de l'entraînement de la turbine principale 8a par l'arbre moteur 7.

De plus, l'air prélevé à travers la deuxième entrée d'air E2 par la turbine annexe 8b peut être refroidi préalablement à son admission à l'intérieur de l'appareil 1a, 1b. A cet effet, l'appareil 1a, 1b peut par exemple être couplé à un système de traitement thermique du flux d'air de refroidissement F2.

Le cloisonnement entre le chemin aéraulique annexe et le chemin aéraulique principal peut être réalisé par tout panneau, support, paroi de forme et de structure adaptée pour permettre la ségrégation optimale entre les flux.

Concernant les sorties d'air S1, S2, elles peuvent être constituées par une ou plusieurs ouvertures formées dans l'embout 20, le caisson 11 et/ou la chemise 19.

Par ailleurs, le nombre de turbines n'est pas limité à deux: des montages en série et/ou en parallèle de plusieurs turbines principales et/ou de plusieurs turbines annexes peuvent être mis en oeuvre.

On notera que l'application d'un appareil 1a, 1b conforme à l'invention à l'assistance respiratoire d'un patient n'est pas restrictive quant à la portée de l'invention. En effet, un appareil 1a, 1b conforme à l'invention au regard de sa structure et/ou de ses modalités de fonctionnement, peut être appliqué à d'autres appareils motorisés de délivrance régulée d'un gaz à système de refroidissement intégré.

Il est encore à noter qu'un appareil 1a, 1b conforme à l'invention peut être utilisé pour traiter de l'air circulant à travers un appareil de conditionnement d'air. L'air acheminé vers l'appareil de conditionnement d'air est formé du flux d'air principal F1 prélevé via la volute 9 d'un appareil 1a, 1b conforme à l'invention. Le flux d'air principal F1 prélevé peut dès lors être enrichi à l'intérieur de la volute 9, par au moins un adjuvant tel qu'un agent d'assainissement de l'appareil de conditionnement d'air et/ou un agent odorant.

## Revendications

1. Appareil (1a, 1b) motorisé d'assistance respiratoire à système de refroidissement intégré, l'appareil (1a, 1b) comportant une enceinte (1) ménageant un compartiment (2) délimité par une chemise (19) logeant un bloc moteur (3), le bloc moteur (3) comportant un carter (4) de réception au moins d'un rotor (6b) et d'un stator (6a) constitutifs d'un moteur (5) d'entraînement d'au moins deux turbines (8a, 8b) montées aux extrémités axiales respectives d'un arbre moteur (7) entraîné par le rotor (6b), lesdites turbines (8a, 8b) se composant au moins d'une turbine principale (8a) génératrice d'un flux d'air principal (F1) dont est issu un gaz patient et d'une turbine annexe (8b) génératrice d'un flux d'air de refroidissement (F2) du moteur (5), l'enceinte (1) ménageant un circuit aéraulique composé au moins de deux chemins aérauliques se composant d'au moins un chemin aéraulique principal d'acheminement du flux d'air principal (F1) et d'au moins un chemin aéraulique annexe d'acheminement du flux d'air de refroidissement (F2) comportant chacun une entrée d'air (E1, E2) des turbines (8a, 8b) vers l'intérieur de l'appareil (1a, 1b) et une sortie d'air (S1, S2) vers l'extérieur de l'appareil (1a, 1b), **caractérisé en ce que** le chemin aéraulique annexe comporte une portion interne (E4) de circulation du flux d'air de refroidissement (F2) provenant de l'entrée d'air correspondante (E2) et s'étendant à l'intérieur du moteur (5) entre le stator (6a) et le rotor (6b), et une portion externe (E3) s'étendant, parallèlement à la portion interne (E4), à l'intérieur d'un espace annulaire (E3) ménagé autour du bloc moteur (3) entre le carter (4) du bloc moteur (3) et la chemise (19) de l'enceinte (1) jusqu'à la sortie d'air correspondante (S2) ménagée dans la chemise (19) du côté de l'entrée d'air de refroidissement (E2), le flux d'air de refroidissement (F2) circulant successivement en sens opposé dans les portions interne (E4) et externe (E3), et **en ce que** le chemin aéraulique principal (E1, E6, S1) et le chemin aéraulique annexe (E2, E4, E5, 22, E3, S2) sont séparés l'un de l'autre par un cloisonnement (18) ménagée entre eux, ledit cloisonnement (18) dissociant la circulation du flux d'air principal (F1) et la circulation du flux d'air de refroidissement (F2) à l'intérieur de l'appareil (1a, 1b).

2. Appareil (1a, 1b) selon la revendication 1, **caractérisé en ce qu'**un volume interfacé entre le bloc moteur (3) et ledit cloisonnement (18) ménage une chambre (27) de collecte et de mélange entre, d'une part de fuites d'air (F3) issues du flux d'air principal (F1) et provenant du chemin aéraulique principal (E1, E6, S1) en traversant le dit cloisonnement (18) et, d'autre part, de fuites d'air (F4) issues du flux d'air de refroidissement (F2) et provenant du bloc moteur (3), et **en ce que** la chambre (27) est en communication aéraulique via au moins un passage d'air (25) avec la portion externe (E3, S2) du chemin aéraulique annexe (E2, E4, E5, 22, E3, S2), de sorte que lesdites fuites d'air (F3, F4) sont drainées hors de la chambre (27) vers l'intérieur de la portion externe (E3, S2) du chemin aéraulique annexe (E2, E4, E5, 22, E3, S2) par le flux d'air de refroidissement (F2) évacué hors du bloc moteur (3), le flux d'air de refroidissement (F2) et lesdites fuites d'air (F3) étant conjointement évacués hors de l'appareil (1a, 1b) via la sortie d'air (S2) du chemin aéraulique annexe (E2, E4, E5, 22, E3, S2).

3. Appareil (1a, 1b) selon l'une quelconque des revendications 1 ou 2, **caractérisé en ce que** le flux d'air de refroidissement (F2) est admis à l'intérieur de la portion interne (E2, E4, E5, 22) du chemin aéraulique annexe (E2, E4, E5, 22, E3, S2) puis est évacué hors du bloc moteur (3) vers la portion externe (E3,S2) du chemin aéraulique annexe à travers au moins une ouverture (22) dite deuxième ouverture traversant la paroi du carter (4) du bloc moteur (3).

4. Appareil (1a, 1b) selon les revendications 2 et 3, **caractérisé en ce que** ladite au moins deuxième ouverture (22) traverse radialement la paroi du carter (4) du bloc moteur (3) et est disposée à faible distance axiale du cloisonnement, et **en ce que** la chambre (27) est axialement (A1) ménagée entre le bloc moteur (3) et le cloisonnement (18) en débouchant axialement (A1) sur la portion externe (E3, S2) du chemin aéraulique annexe (E2, E4, E5, 22, E3, S2).

5. Appareil (1a, 1b) selon l'une quelconque des revendications 1 à 4, **caractérisé en ce que** des organes d'étanchéité (24a, 24b, 24c) sont montés autour de l'arbre moteur (7) avec un jeu minimal, et sont composés au moins d'un organe d'étanchéité (24a) solidaire du cloisonnement (18) du côté d'une volute (9), et d'organes d'étanchéité (24b, 24c) solidaires de paliers de roulement (23) montés sur le carter (4) hors du bloc moteur (3).

6. Appareil (1a, 1b) selon l'une quelconque des revendications 1 à 5, **caractérisé en ce que** la turbine annexe (8b) est logée à l'intérieur d'une cavité (E4) d'admission du flux d'air de refroidissement (F2) vers l'intérieur du bloc moteur, via l'entrée d'air (E2) du chemin aéraulique annexe (E2, E4, E5, 22, E3, S2), la cavité (E4) étant isolée de la portion externe (E3,S2) du chemin aéraulique annexe (E2, E4, E5, 22, E3, S2) et étant ouverte sur l'intérieur du bloc moteur (3) via au moins une autre ouverture (13) dite première ouverture ménagée à travers le carter (4).

7. Appareil (1a) selon les revendications 3 et 6, **caractérisé en ce que** la cavité (E4) est délimitée entre la paroi périphérique de la chemise (19) et une couronne (26) entourant radialement le carter (4) du bloc moteur (3), la sortie d'air (S2) du chemin aéraulique annexe (E2, E4, E5, 22, E3, S2) étant ménagée radialement à travers la chemise (19) en interposition axiale entre la couronne (26) et ladite au moins deuxième ouverture (22) traversant la paroi du carter (4) du bloc moteur (3).

8. Appareil (1b) selon la revendication 6, **caractérisé en ce que** la cavité (E4) est ménagée par le volume intérieur d'un caisson (11) qui est monté axialement sur le carter (4) et qui ménage l'entrée d'air (E2) du chemin aéraulique annexe (E2, E4, E5, 22, E3, S2), le caisson (11) étant disposé à l'intérieur du compartiment (2) recevant le bloc moteur (3) en prolongement axial du carter (4).

9. Appareil (1b) selon la revendication 8, **caractérisé en ce que** le caisson (11) est configuré en cône dont le plus grand débouché est orienté vers le moteur (5) et dont le plus petit débouché est prolongé axialement par un conduit d'entrée d'air (12) intégré au caisson (11), le conduit d'entrée d'air (12) traversant axialement par emboîtement une paroi de la chemise (19) en ménageant l'entrée d'air (E2) du chemin aéraulique annexe (E2, E4, E5, 22, E3, S2) et en formant un organe de centrage axial du bloc moteur (3) à l'intérieur du compartiment (2).

10. Appareil (1a, 1b) selon l'une quelconque des revendications 1 à 9, **caractérisé en ce que** la sortie d'air (S2) du chemin aéraulique annexe est ménagée par un débouché vers l'extérieur de l'enceinte (1) de conduit de sortie d'air (16) intégré à la chemise (19).

11. Appareil (1b) selon les revendications 9 et 10, **caractérisé en ce que** le conduit d'entrée d'air (12) et le conduit de sortie d'air (16) du chemin aéraulique annexe (E2, E4, E5, 22, E3, S2) sont disposés à une première extrémité axiale (17a) de l'appareil opposée à son autre deuxième extrémité axiale (17b) à laquelle le chemin aéraulique principal (E1, E6, S1) est ménagé entre l'entrée d'air (E1) et la sortie d'air (E2) qu'il comporte.

12. Appareil (1a, 1b) selon l'une quelconque des revendications 1 à 11, **caractérisé en ce que** l'enceinte (1) comprend une volute (9) logeant la turbine principale (8a) et délimitant le chemin aéraulique principal (E1, E6, S1), ladite volute (9) ménageant l'entrée d'air (E1) et la sortie d'air (S1) du chemin aéraulique principal et étant pourvue d'au moins un organe de raccordement (10) à un conduit d'admission d'un adjuvant à l'intérieur de la volute (9).

13. Appareil (1a, 1b) selon la revendication 12, **caractérisé en ce qu'**au moins une paroi délimitant la volute (9) ménage le cloisonnement (18) de séparation entre le chemin aéraulique principal (E1, E6, S1) et le chemin aéraulique annexe (E2, E4, E5, 22, E3, S2), ladite paroi ménageant le cloisonnement (18) incorporant ledit organe de raccordement (10) et délimitant entre eux d'une part la volute (9) et la sortie d'air (S1) du flux d'air principal (F1) hors de l'appareil qui est incorporée à la volute (9) et d'autre part le compartiment (2) de réception du bloc moteur (3).

## Patentansprüche

1. Motorisierte Atmungshilfsvorrichtung (1a, 1b) mit integriertem Kühlsystem, wobei die Vorrichtung (1a, 1b) eine Umschließung (1) aufweist, in der ein von einem Mantel (19) begrenzter Raum (2) ausgebildet ist, in dem ein Motorblock (3) untergebracht ist, wobei der Motorblock (3) ein Gehäuse (4) zur Aufnahme wenigstens eines Rotors (6b) und eines Stators (6a) aufweist, die Bestandteile eines Motors (5) zum Antrieb von wenigstens zwei Turbinen (8a, 8b) sind, die an den jeweiligen axialen Enden einer von dem Rotor (6b) angetriebenen Motorwelle (7) angebracht sind, wobei die Turbinen (8a, 8b) aus wenigstens einer Hauptturbine (8a), die einen Hauptluftstrom (F1) erzeugt, von dem ein Patientengas ausgeht, und aus einer Nebenturbine (8b), die einen Kühlluftstrom (F2) für den Motor (5) erzeugt, bestehen, wobei in der Umschließung (1) ein lufttechnischer Kreislauf ausgebildet ist, der aus wenigstens zwei lufttechnischen Wegen besteht, die aus wenigstens einem lufttechnischen Hauptweg zur Lenkung des Hauptluftstroms (F1) und wenigstens einem lufttechnischen Nebenweg zur Lenkung des Kühlluftstroms (F2) bestehen, die jeweils einen Lufteinlass (E1, E2) der Turbinen (8a, 8b) zum Inneren der Vorrichtung (1a, 1b) hin und einen Luftauslass (S1, S2) zur Außenseite der Vorrichtung (1a, 1b) hin aufweisen, **dadurch gekennzeichnet, dass** der lufttechnische Nebenweg einen inneren Abschnitt (E4) der Strömung des Kühlluftstroms (F2) aufweist, der vom entsprechenden Lufteinlass (E2) kommt und sich im Inneren des Motors (5) zwischen dem Stator (6a) und dem Rotor (6b) erstreckt, und einen äußeren Abschnitt (E3), der sich parallel zum inneren Abschnitt (E4) im Inneren eines Ringraumes (E3) erstreckt, der um den Motorblock (3) herum zwischen dem Gehäuse (4) des Motorblocks (3) und dem Mantel (19) der Umschließung (1) bis zu dem entsprechenden Luftauslass (S2) ausgebildet ist, der im Mantel (19) auf der Seite des Kühllufteinlasses (E2) ausgebildet ist, wobei der Kühlluftstrom (F2) nacheinander in entgegengesetzter Richtung im inneren (E4) und äußeren Abschnitt (E3) strömt, und dadurch, dass der lufttechnische Hauptweg (E1, E6, S1) und der lufttechnische Nebenweg (E2, E4, E5, 22, E3, S2) durch eine Abschottung (18) voneinander getrennt sind, die zwischen ihnen ausgebildet ist, wobei die Abschottung (18) die Strömung des Hauptluftstroms (F1) und die Strömung des Kühlluftstroms (F2) im Inneren der Vorrichtung (1a, 1b) trennt.

2. Vorrichtung (1a, 1b) nach Anspruch 1, **dadurch gekennzeichnet, dass** in einem Volumen, das eine Schnittstelle zwischen dem Motorblock (3) und der Abschottung (18) bildet, eine Kammer (27) zum Sammeln und zur Mischung zwischen einerseits Leckluftströmen (F3), die vom Hauptluftstrom (F1) ausgehen und vom lufttechnischen Hauptweg (E1, E6, S1) kommen, wobei sie die Abschottung (18) durchqueren, und andererseits Leckluftströmen (F4), die vom Kühlluftstrom (F2) ausgehen und vom Motorblock (3) kommen, ausgebildet ist, und dadurch, dass die Kammer (27) über wenigstens einen Luftdurchlass (25) mit dem äußeren Abschnitt (E3, S2) des lufttechnischen Nebenweges (E2, E4, E5, 22, E3, S2) in lufttechnischer Verbindung steht, so dass die Leckluftströme (F3, F4) durch den Kühlluftstrom (F2), der aus dem Motorblock (3) abgeführt wird, aus der Kammer (27) in Richtung des Inneren des äußeren Abschnitts (E3, S2) des lufttechnischen Nebenweges (E2, E4, E5, 22, E3, S2) abgezogen werden, wobei der Kühlluftstrom (F2) und die Leckluftströme (F3) zusammen über den Luftauslass (S2) des lufttechnischen Nebenweges (E2, E4, E5, 22, E3, S2) aus der Vorrichtung (1a, 1b) abgeführt werden.

3. Vorrichtung (1a, 1b) nach einem der Ansprüche 1 oder 2, **dadurch gekennzeichnet, dass** der Kühlluftstrom (F2) ins Innere des inneren Abschnitts (E2, E4, E5, 22) des lufttechnischen Nebenweges (E2, E4, E5, 22, E3, S2) eingelassen wird und danach aus dem Motorblock (3) in Richtung des äußeren Abschnitts (E3, S2) des lufttechnischen Nebenweges durch wenigstens eine Öffnung (22), zweite Öffnung genannt, welche die Wand des Gehäuses (4) des Motorblocks (3) durchquert, abgeführt wird.

4. Vorrichtung (1a, 1b) nach den Ansprüchen 2 und 3, **dadurch gekennzeichnet, dass** die wenigstens eine zweite Öffnung (22) die Wand des Gehäuses (4) des Motorblocks (3) radial durchquert und in geringem axialem Abstand von der Abschottung angeordnet ist, und dadurch, dass die Kammer (27) axial (A1) zwischen dem Motorblock (3) und der Abschottung (18) ausgebildet ist, wobei sie axial (A1) am äußeren Abschnitt (E3, S2) des lufttechnischen Nebenweges (E2, E4, E5, 22, E3, S2) mündet.

5. Vorrichtung (1a, 1b) nach einem der Ansprüche 1 bis 4, **dadurch gekennzeichnet, dass** Abdichtungsorgane (24a, 24b, 24c) mit einem minimalen Spiel um die Motorwelle (7) herum angebracht sind und aus wenigstens einem mit der Abschottung (18) fest verbundenen Abdichtungsorgan (24a) auf der Seite eines Spiralgehäuses (9) und Abdichtungsorganen (24b, 24c), die mit am Gehäuse (4) außerhalb des Motorblocks (3) angebrachten Wälzlagern (23) fest verbunden sind, bestehen.

6. Vorrichtung (1a, 1b) nach einem der Ansprüche 1 bis 5, **dadurch gekennzeichnet, dass** die Nebenturbine (8b) im Inneren eines Hohlraums (E4) zum Einlassen des Kühlluftstroms (F2) in Richtung des Inneren des Motorblocks über den Lufteinlass (E2) des lufttechnischen Nebenweges (E2, E4, E5, 22, E3, S2) untergebracht ist, wobei der Hohlraum (E4) vom äußeren Abschnitt (E3, S2) des lufttechnischen Nebenweges (E2, E4, E5, 22, E3, S2) isoliert ist und zum Inneren des Motorblocks (3) über mindestens eine weitere Öffnung (13), erste Öffnung genannt, die durch das Gehäuse (4) hindurch ausgebildet ist, geöffnet ist.

7. Vorrichtung (1a) nach den Ansprüchen 3 und 6, **dadurch gekennzeichnet, dass** der Hohlraum (E4) zwischen der Umfangswand des Mantels (19) und einem Kranz (26), der das Gehäuse (4) des Motorblocks (3) radial umgibt, begrenzt wird, wobei der Luftauslass (S2) des lufttechnischen Nebenweges (E2, E4, E5, 22, E3, S2) radial durch den Mantel (19) hindurch ausgebildet ist, wobei er axial zwischen dem Kranz (26) und der wenigstens einen zweiten Öffnung (22), welche die Wand des Gehäuses (4) des Motorblocks (3) durchquert, positioniert ist.

8. Vorrichtung (1b) nach Anspruch 6, **dadurch gekennzeichnet, dass** der Hohlraum (E4) vom Innenvolumen eines Behälters (11) gebildet wird, welcher axial am Gehäuse (4) angebracht ist und an welchem der Lufteinlass (E2) des lufttechnischen Nebenweges (E2, E4, E5, 22, E3, S2) ausgebildet ist, wobei der Behälter (11) im Inneren des Raumes (2), der den Motorblock (3) aufnimmt, in der axialen Verlängerung des Gehäuses (4) angeordnet ist.

9. Vorrichtung (1b) nach Anspruch 8, **dadurch gekennzeichnet, dass** der Behälter (11) als Kegel gestaltet ist, dessen größere Mündung zum Motor (5) hin gerichtet ist und dessen kleinere Mündung axial durch eine Lufteinlassleitung (12) verlängert wird, die in den Behälter (11) integriert ist, wobei die Lufteinlassleitung (12) eine Wand des Mantels (19) durch Einstecken axial durchquert, wobei sie den Lufteinlass (E2) des lufttechnischen Nebenweges (E2, E4, E5, 22, E3, S2) ausbildet und wobei sie ein Organ zur axialen Zentrierung des Motorblocks (3) im Inneren des Raumes (2) bildet.

10. Vorrichtung (1a, 1b) nach einem der Ansprüche 1 bis 9, **dadurch gekennzeichnet, dass** der Luftauslass (S2) des lufttechnischen Nebenweges von einer Mündung einer in den Mantel (19) integrierten Luftauslassleitung (16) zur Außenseite der Umschließung (1) hin gebildet wird.

11. Vorrichtung (1b) nach den Ansprüchen 9 und 10, **dadurch gekennzeichnet, dass** die Lufteinlassleitung (12) und die Luftauslassleitung (16) des lufttechnischen Nebenweges (E2, E4, E5, 22, E3, S2) an einem ersten axialen Ende (17a) der Vorrichtung angeordnet sind, das ihrem anderen, zweiten axialen Ende (17b) gegenüberliegt, an welchem der lufttechnische Hauptweg (E1, E6, S1) zwischen dem Lufteinlass (E1) und dem Luftauslass (E2), welche er aufweist, ausgebildet ist.

12. Vorrichtung (1a, 1b) nach einem der Ansprüche 1 bis 11, **dadurch gekennzeichnet, dass** die Umschließung (1) ein Spiralgehäuse (9) umfasst, in dem die Hauptturbine (8a) untergebracht ist und das den lufttechnischen Hauptweg (E1, E6, S1) begrenzt, wobei das Spiralgehäuse (9) den Lufteinlass (E1) und den Luftauslass (S1) des lufttechnischen Hauptweges bildet und mit wenigstens einem Anschlussorgan (10) für eine Leitung zum Einlassen eines Zusatzmittels ins Innere des Spiralgehäuses (9) versehen ist.

13. Vorrichtung (1a, 1b) nach Anspruch 12, **dadurch gekennzeichnet, dass** wenigstens eine Wand, die das Spiralgehäuse (9) begrenzt, die Abschottung (18) zur Trennung zwischen dem lufttechnischen Hauptweg (E1, E6, S1) und dem lufttechnischen Nebenweg (E2, E4, E5, 22, E3, S2) bildet, wobei in die Wand, welche die Abschottung (18) bildet, das Anschlussorgan (10) integriert ist, und wobei sie einerseits das Spiralgehäuse (9) und den Luftauslass (S1) des Hauptluftstroms (F1) aus der Vorrichtung, welcher in das Spiralgehäuse (9) integriert ist, und andererseits den Raum (2) zur Aufnahme des Motorblocks (3) voneinander abgrenzt.

## Claims

1. Motorized respiratory assistance device (1a, 1b) with integrated cooling system, the device (1a, 1b) comprising an enclosure (1) forming a compartment (2) delimited by a jacket (19) housing a motor block (3), the motor block (3) comprising a casing (4) receiving at least a rotor (6b) and a stator (6a) forming a motor (5) driving at least two turbines (8a, 8b) mounted at the respective axial ends of a motor shaft (7) driven by the rotor (6b), said turbines (8a, 8b) being composed of at least one main turbine (8a) generating a main air flow (F1) from which is derived a patient gas, and a secondary turbine (8b) generating a cooling air flow (F2) for the motor (5), the enclosure (1) forming an aeraulic circuit composed at least of two aeraulic paths composed of at least one main aeraulic path conveying the main air flow (F1) and at least one secondary aeraulic path conveying the cooling air flow (F2), each comprising an air inlet (E1, E2) for the turbines (8a, 8b) to the inside of the device (1a, 1b) and an air outlet (S1, S2) to the outside of the device (1a, 1b), **characterized in that** the secondary aeraulic path comprises an inner portion (E4) for circulation of the cooling air flow (F2) originating from the corresponding air inlet (E2) and extending inside the motor (5) between the stator (6a) and the rotor (6b), and an outer portion (E3) extending, parallel to the inner portion (E4), inside an annular space (E3) formed around the motor block (3) between the casing (4) of the motor block (3) and the jacket (19) of the enclosure (1) to the corresponding air outlet (S2) formed in the jacket (19) on the side of the cooling air inlet (E2), the cooling air flow (F2) circulating successively in opposite directions in the inner (E4) and outer (E3) portions, and **in that** the main aeraulic path (E1, E6, S1) and the secondary aeraulic path (E2, E4, E5, 22, E3, S2) are separated from one another by a partitioning (18) formed between them, said partitioning (18) separating the circulation of the main air flow (F1) and the circulation of the cooling air flow (F2) inside the device (1a, 1b).

2. The device (1a, 1b) as claimed in claim 1, **characterized in that** a volume interfaced between the motor block (3) and said partitioning (18) forms a chamber (27) for collecting and mixing between, on the one hand, air leaks (F3) from the main air flow (F1) and originating from the main aeraulic path (E1, E6, S1) by passing through said partitioning (18) and, on the other hand, air leaks (F4) from the cooling air flow (F2) and originating from the motor block (3), and **in that** the chamber (27) is in aeraulic communication via at least one air passage (25) with the outer portion (E3, S2) of the secondary aeraulic path (E2, E4, E5, 22, E3, S2), such that said air leaks (F3, F4) are drained out of the chamber (27) to the inside of the outer portion (E3, S2) of the secondary aeraulic path (E2, E4, E5, 22, E3, S2) by the cooling air flow (F2) discharged out of the motor block (3), the cooling air flow (F2) and said air leaks (F3) being jointly discharged out of the device (1a, 1b) via the air outlet (S2) of the secondary aeraulic path (E2, E4, E5, 22, E3, S2).

3. The device (1a, 1b) as claimed in either one of claims 1 and 2, **characterized in that** the cooling air flow (F2) is admitted into the inner portion (E2, E4, E5, 22) of the secondary aeraulic path (E2, E4, E5, 22, E3, S2) then is discharged out of the motor block (3) to the outer portion (E3,S2) of the secondary aeraulic path through at least one aperture (22), called second aperture, passing through the wall of the casing (4) of the motor block (3).

4. The device (1a, 1b) as claimed in claims 2 and 3, **characterized in that** said at least second aperture (22) passes radially through the wall of the casing (4) of the motor block (3) and is disposed at a short axial distance from the partitioning, and **in that** the chamber (27) is axially (A1) formed between the motor block (3) and the partitioning (18) by emerging axially (A1) on the outer portion (E3, S2) of the secondary aeraulic path (E2, E4, E5, 22, E3, S2).

5. The device (1a, 1b) as claimed in any one of claims 1 to 4, **characterized in that** sealing members (24a, 24b, 24c) are mounted around the motor shaft (7) with a minimal play, and are composed at least of a sealing member (24a) secured to the partitioning (18) on the side of a volute (9), and of sealing members (24b, 24c) secured to rolling bearings (23) mounted on the casing (4) outside of the motor block (3).

6. The device (1a, 1b) as claimed in any one of claims 1 to 5, **characterized in that** the secondary turbine (8b) is housed inside a cavity (E4) for the intake of the cooling air flow (F2) to the inside of the motor block, via the air inlet (E2) of the secondary aeraulic path (E2, E4, E5, 22, E3, S2), the cavity (E4) being isolated from the outer portion (E3,S2) of the secondary aeraulic path (E2, E4, E5, 22, E3, S2) and being open on the inside of the motor block (3) via at least one other aperture (13), called first aperture, formed through the casing (4).

7. The device (1a) as claimed in claims 3 and 6, **characterized in that** the cavity (E4) is delimited between the peripheral wall of the jacket (19) and a crown ring (26) radially surrounding the casing (4) of the motor block (3), the air outlet (S2) of the secondary aeraulic path (E2, E4, E5, 22, E3, S2) being formed radially through the jacket (19) by axial interposition between the crown ring (26) and said at least second aperture (22) passing through the wall of the casing (4) of the motor block (3).

8. The device (1b) as claimed in claim 6, **characterized in that** the cavity (E4) is formed by the internal volume of a caisson (11) which is mounted axially on the casing (4) and which forms the air inlet (E2) of the secondary aeraulic path (E2, E4, E5, 22, E3, S2), the caisson (11) being disposed inside the compartment (2) receiving the motor block (3) in axial extension of the casing (4).

9. The device (1b) as claimed in claim 8, **characterized in that** the caisson (11) is configured in cone-form, the largest outlet of which is oriented toward the motor (5) and the smallest outlet of which is prolonged axially by an air inlet duct (12) incorporated in the caisson (11), the air inlet duct (12) passing axially by fit through a wall of the jacket (19), forming the air inlet (E2) of the secondary aeraulic path (E2, E4, E5, 22, E3, S2) and forming an axial centering member of the motor block (3) inside the compartment (2).

10. The device (1a, 1b) as claimed in any one of claims 1 to 9, **characterized in that** the air outlet (S2) of the secondary aeraulic path is formed by an outward emergence from the enclosure (1) of the air outlet duct (16) incorporated in the jacket (19).

11. The device (1b) as claimed in claims 9 and 10, **characterized in that** the air inlet duct (12) and the air outlet duct (16) of the secondary aeraulic path (E2, E4, E5, 22, E3, S2) are disposed at a first axial end (17a) of the device opposite its second axial end (17b) at which the main aeraulic path (E1, E6, S1) is formed between the air inlet (E1) and the air outlet (E2) that it comprises.

12. The device (1a, 1b) as claimed in any one of claims 1 to 11, **characterized in that** the enclosure (1) comprises a volute (9) housing the main turbine (8a) and delimiting the main aeraulic path (E1, E6, S1), said volute (9) forming the air inlet (E1) and the air outlet (S1) of the main aeraulic path and being provided with at least one connecting member (10) to a duct for the intake of an additive to the inside of the volute (9).

13. The device (1a, 1b) as claimed in claim 12, **characterized in that** at least one wall delimiting the volute (9) forms the separating partitioning (18) between the main aeraulic path (E1, E6, S1) and the secondary aeraulic path (E2, E4, E5, 22, E3, S2), said wall forming the partitioning (18) incorporating said connecting member (10) and delimiting between them, on the one hand, the volute (9) and the air outlet (S1) for the main air flow (F1) out of the device which is incorporated in the volute (9) and, on the other hand, the compartment (2) receiving the motor block (3).
